# FASCICULE DE BREVET EUROPEEN

(11) **EP 1 516 590 B1**
(45) Date de publication et mention de la délivrance du brevet: **14.06.2006**
(21) Numéro de dépôt: 04292202.1
(22) Date de dépôt: 14.09.2004
(51) Int. Cl.: A61B 17/16

(54) **Dispositif pour râper le canal intra-médullaire d'un os et système pour mettre en oeuvre ce dispositif**
Vorrichtung zum Raspeln des Knochenmarkraumes und Vorrichtung zu dessen Herstellung
Device for rasping the bone canal and its manufacturing assembly

(30) Priorité: 16.09.2003 FR 0310861
(43) Date de publication de la demande: 23.03.2005
(73) Titulaire: A.T.S. SFERIC S.A.S., 41500 Menars (FR)
(72) Inventeur: Grimard, Jean-Christophe, 41120 Cellettes (FR); Cousin, Thierry, 41500 Menars (FR); Costa, José, 92130 Issy les Moulineaux (FR)
(74) Mandataire: Debay, Yves

(56) Documents cités:
- EP-A- 0 563 585
- EP-A- 1 174 201
- US-A- 5 527 316

## Description

La présente invention concerne un dispositif pour râper le canal intra-médullaire d'un os. L'invention concerne également un système permettant de mettre en oeuvre le dispositif.

En arthroplastie articulaire, et quelle que soit l'articulation, il existe plusieurs types de prothèses qui diffèrent selon la pathologie à traiter, l'état de santé du patient, le type de voie et de technique opératoire.

Les chirurgiens souhaitent adapter la préparation du canal intra-médullaire de l'os en fonction du type de prothèse qu'il souhaite poser, afin d'assurer un geste opératoire efficace et sans risque, tout en garantissant la fiabilité de la fixation de l'implant.

Le calibrage médullaire est effectué à l'aide d'une râpe. Celle-ci façonne le canal intra-médullaire avec précision pour que le volume obtenu soit en correspondance avec le volume de l'implant à mettre en place. Pour cela, la râpe est couverte sur son enveloppe extérieure partiellement ou en totalité d'une denture qui va couper et tasser l'os spongieux et cortical.

Il existe différents types de dentures, plus ou moins agressives, qui permettent le calibrage médullaire souhaité par le chirurgien, en fonction du type de prothèse choisi. Pour ces différents types de denture, il est possible de faire varier les paramètres des dents : angle de coupe, plat en sommet de dent, pas, orientation des dents.

Il est connu par le brevet EP 0 634 145 une râpe en forme de tige creuse munie de dents sur sa surface. La râpe comporte une fente longitudinale procurant une élasticité à la râpe. Un inconvénient de cette râpe est que les contraintes se concentrent au niveau du fond des dents lors du râpage de l'os, entraînant des risques importants de casse.

Ce problème est résolu dans le brevet EP 0 563 585 (le préambule de la revendication 1 est basé sur ce document) et la demande de brevet EP 1 174 201 par une denture en hélice permettant de répartir les contraintes sur l'ensemble de l'hélice formée par la denture.

Le brevet EP 0 563 585 enseigne une râpe en plastique munie d'une denture en hélice avec différents angles d'attaque le long de la râpe, la denture étant éventuellement dédoublée sur la tranche de la râpe. La râpe comporte un canal intérieur permettant l'aspiration des débris d'os lors du râpage.

La demande de brevet EP 1 174 201 enseigne une râpe munie d'une denture disposée suivant une hélice, qui est usinée dans la surface du corps de la râpe.

Un inconvénient de l'ensemble de ces râpes est qu'elles sont onéreuses à fabriquer, donc utilisées pour plusieurs patients, avec un nettoyage et une stérilisation entre deux patients. Toutefois, le nettoyage des râpes est difficile du fait des formes anguleuses des dentures, et en particulier des zones de rétention que constituent les dents, ce qui peut entraîner une contamination d'os ou de moelle d'un patient à un autre et donc de transmission de maladies type Kreuzfeld-Jacob.

La présente invention a pour but de pallier certains inconvénients de l'art antérieur en proposant une râpe facile à nettoyer et ne comportant pas de risque de contamination d'un patient à un autre.

Ce but est atteint par un dispositif pour râper le canal intra-médullaire d'un os, comprenant un corps allongé selon un axe sensiblement parallèle à l'axe longitudinal du canal et des éléments tranchants, le corps allongé ayant une section transversale variable et une forme apte à coopérer avec la paroi interne du canal intra-médullaire de l'os, caractérisé en ce que les éléments tranchants sont disposés sur un bandeau amovible et jetable enroulé autour du corps allongé suivant une rainure hélicoïdale formée sur ledit corps.

Selon une autre particularité, la rainure est un filetage arrondi.

Selon une autre particularité, le bandeau est souple, de façon à épouser la forme de la rainure, et en ce qu'il comprend au moins un ergot formé à proximité d'une extrémité du bandeau, ledit ergot coopérant avec une gorge formée à proximité de l'extrémité supérieure de la rainure pour maintenir le bandeau en haut du corps.

Selon une particularité, le bandeau comprend un deuxième ergot formé à proximité de l'autre extrémité du bandeau, ledit deuxième ergot coopérant avec une deuxième gorge formée à proximité de l'extrémité inférieure de la rainure pour maintenir le bandeau en bas du corps.

Selon une particularité, un manchon est vissé sur l'extrémité inférieure du corps pour maintenir le bandeau en bas du corps.

Selon une particularité, le bandeau est collé sur le corps grâce à une colle biocompatible.

Selon une particularité, le bandeau est maintenu sur le corps par un abrasif fixé sous le bandeau.

Selon une autre particularité, le bandeau est un ressort vissé dans la rainure.

Selon une autre particularité, la largeur du bandeau est, au maximum, égale au pas de l'hélice, de façon à ce que, une fois que le bandeau est rendu solidaire du corps, les bords du bandeau soient, au maximum, jointifs.

Selon une autre particularité, les éléments tranchants du bandeau sont constitués d'une ou plusieurs lignes de dents.

Selon une autre particularité, les éléments tranchants du bandeau sont constitués d'une ou plusieurs arêtes, de longueur sensiblement égale à la longueur du bandeau, chacune arête au moins un plan de coupe.

Un autre but de l'invention est de proposer un système de montage du bandeau sur le corps du dispositif.

Ce but est atteint par un système caractérisé en ce qu'il comprend un axe sensiblement horizontal pouvant tourner sur lui-même et mobile le long de son axe, sur lequel sont montés, l'un derrière l'autre, le corps du dispositif, ainsi qu'un corps d'un deuxième dispositif servant de gabarit, les axes longitudinaux du corps du dispositif et du gabarit étant sensiblement alignés avec ledit axe, et en ce qu'il comprend également une roue sur laquelle est enroulé le bandeau, ainsi qu'un doigt, monté fixe en translation par rapport à la roue et engagé dans le filetage du gabarit, la distance séparant le plan de la roue et l'axe du doigt étant sensiblement égale à la distance séparant l'extrémité inférieure du corps du dispositif de celle du gabarit, de telle sorte que, au fur et à mesure que le gabarit et le corps du dispositif subissent une rotation autour de l'axe du système de montage, le doigt se déplace le long du filetage du gabarit et permet que le bandeau s'enroule correctement dans le filetage du corps du dispositif.

D'autres particularités et avantages de la présente invention apparaîtront plus clairement à la lecture de la description ci-après, faite en référence aux dessins annexés, dans lesquels :
- la figure 1 représente une vue de côté de la râpe sans le bandeau amovible,
- la figure 2 représente une vue de détail en coupe longitudinale de la râpe munie du bandeau amovible selon un mode de réalisation,
- la figure 3 représente une vue en perspective du bandeau amovible selon le mode de réalisation de la figure 2,
- la figure 4 représente une vue de côté du système de montage du bandeau sur la râpe,
- les figures 5 et 6 représentent une vue en coupe transversale du bandeau selon un deuxième, respectivement troisième, mode de réalisation.

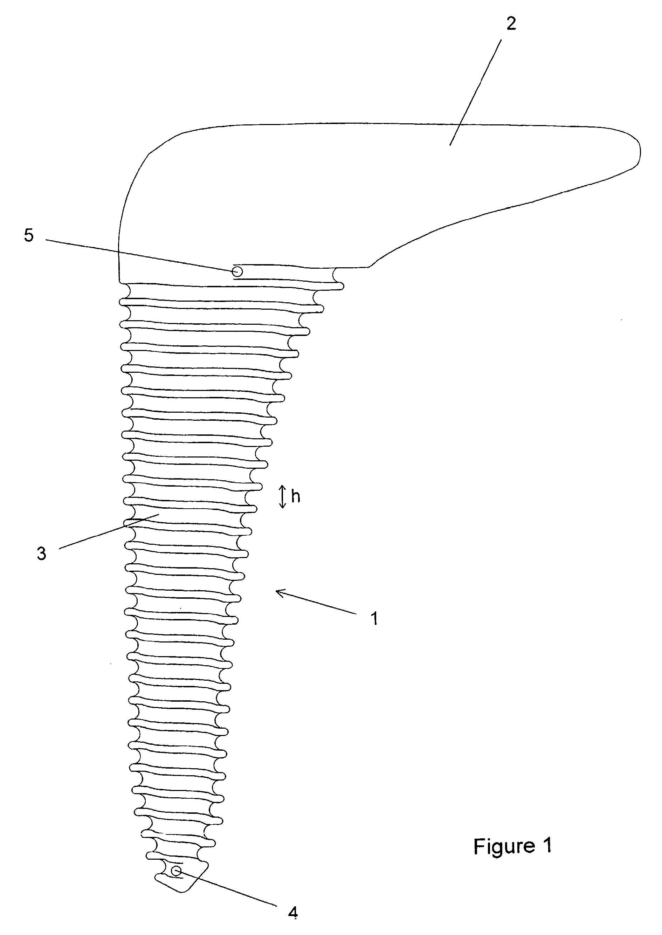

La râpe selon l'invention, représentée en particulier sur la figure 1, est constituée d'un corps (1) et d'une poignée (2). La poignée (2) est par exemple soudée ou amovible. Dans le cas où elle est amovible, elle peut être fixée à l'extrémité supérieure du corps de la râpe par exemple par encliquetage. Le système d'encliquetage de la râpe peut également recevoir une tête d'essai.

Le corps (1) de la râpe est allongé selon un axe sensiblement parallèle à l'axe longitudinal du canal intra-médullaire de l'os à râper. Il a une section transversale variable lui permettant de collaborer avec la paroi interne du canal intra-médullaire de l'os.

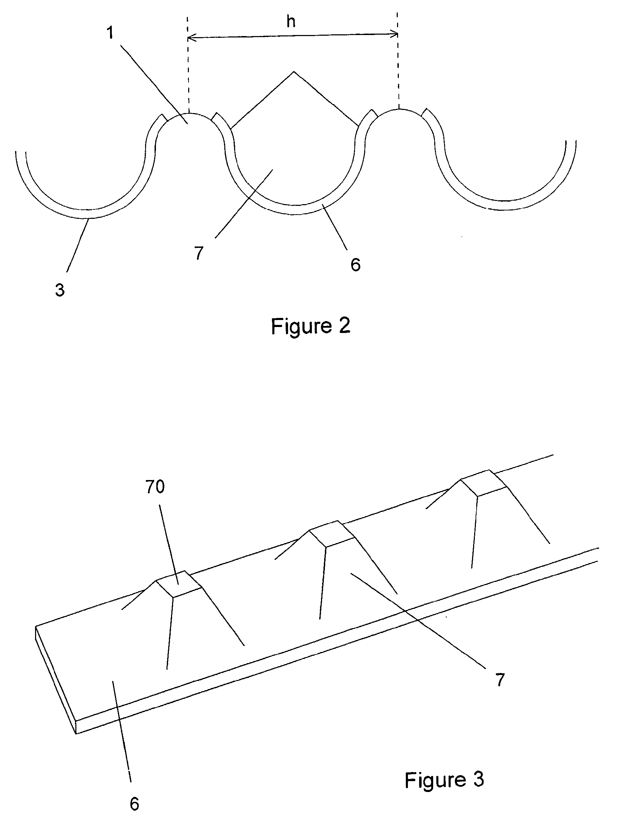

Un filetage (3) en hélice est formé sur le corps (1) de la râpe selon l'invention, permettant d'accueillir un bandeau (6) amovible muni d'éléments tranchants (7). Ce bandeau (6), représenté en particulier sur les figures 3, 5 et 6, est réalisé en matériau souple, de façon à pouvoir s'enrouler autour du corps (1) de la râpe. En revanche, ie corps (1) de ia râpe est rigide, par exemple en métal, de façon à bien maîtriser le râpage. Les éléments tranchants (7) sont réalisés par exemple par repoussage ou usinage. Les éléments tranchants (7) sont constitués, soit d'une ou plusieurs arêtes parallèles entre elles, chacune de longueur sensiblement égale à la longueur du bandeau, soit d'une ou plusieurs lignes de dents, sur toute la longueur du bandeau. Dans ce dernier cas, les caractéristiques géométriques des dents sont choisies en fonction du type d'os à râper ou du type de calibrage recherché.

Le bandeau (6) est monté sur le corps (1) de la râpe avant l'intervention chirurgicale, et démonté puis jeté après l'intervention. Le corps (1) de la râpe est réutilisé après nettoyage et stérilisation, puis montage d'un nouveau bandeau. Le corps de la râpe est lisse et le filetage (3) est arrondi, comme représenté sur la figure 2, ce qui permet un nettoyage aisé, et élimine le risque que des débris d'os, de sang ou de moelle restent accrochés sur le corps (1) de la râpe lorsque cette dernière est utilisée pour un nouveau patient.

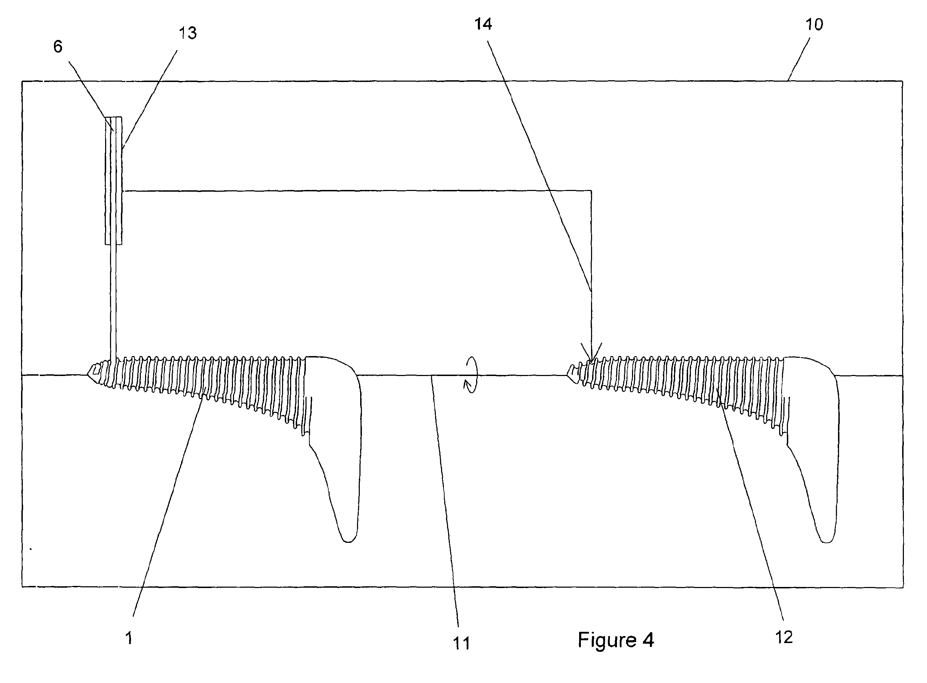

Le bandeau (6) est monté dans le filetage (3) du corps (1) de la râpe, soit manuellement, soit à l'aide d'un système de montage (10), représenté en particulier sur la figure 4. Ce système de montage (10) comprend un axe (11) sensiblement horizontal pouvant tourner sur lui-même et mobile le long de son axe. Le corps (1) de la râpe sur lequel doit être monté un bandeau, ainsi qu'un corps de râpe servant de gabarit (12), sont montés l'un derrière l'autre sur cet axe (11), de façon à ce que l'axe longitudinal des corps de râpe soient sensiblement alignés avec ledit axe (11). Le bandeau (6) est enroulé sur une roue (13) située au-dessus du corps (1) de la râpe sur lequel doit être monté le bandeau (6). Un doigt (14), monté fixe en translation par rapport à la roue (13), est engagé dans le filetage du gabarit (12), de telle façon que la distance séparant le plan de la roue (13) et l'axe du doigt soit sensiblement égale à la distance séparant l'extrémité inférieure du corps (1) de la râpe sur lequel doit être monté le bandeau (6) et celle du gabarit (12). Ainsi, au fur et à mesure que le gabarit (12) et le corps (1) de la râpe subissent une rotation autour de l'axe (11) du système de montage (10), ie doigt (14) se déplace le long du filetage du gabarit (12) et permet que le bandeau (6) s'enroule correctement dans le filetage du corps (1) de la râpe.

Le montage du bandeau (6) sur le corps (1) de la râpe débute par l'encliquetage d'un premier ergot (non représenté) du bandeau dans une gorge (4, 5) située à proximité de l'extrémité inférieure, respectivement supérieure, du filetage (3), suivant le sens dans lequel le montage du bandeau est réalisé (de bas en haut ou de haut en bas du corps de la râpe). Après que le bandeau (6) ait été monté dans le filetage (3), un deuxième ergot (non représenté) est encliqueté dans une deuxième gorge (5, 4) située à proximité de l'extrémité supérieure, respectivement inférieure, du filetage (3).

Dans une variante non représentée, le corps de la râpe ne comprend pas de gorge inférieure ; pour maintenir l'extrémité inférieure du bandeau sur le corps de la râpe, un manchon est vissé sur son extrémité inférieure.

Dans un premier mode de réalisation, le bandeau (6) est maintenu dans le filetage (3) par collage grâce à une colle biocompatible destructible en chauffant à température relativement peu élevée, de façon à ne pas endommager le corps de la râpe, ou avec du dissolvant n'ayant pas d'effet sur le corps (1) de la râpe.

Dans un deuxième mode de réalisation, le bandeau (6) est maintenu dans le filetage (3) grâce à la présence d'un abrasif fixé sous le bandeau (6). Ce mode de réalisation présente le désavantage de s'user plus rapidement du fait des marques laissées par l'abrasif sur le corps de la râpe.

Dans ces deux premiers modes de réalisation, le bandeau est monté sur le corps de la râpe grâce au système de montage (10) de la présente invention.

Dans un troisième mode de réalisation, le bandeau est métallique et élastique et se comporte comme un ressort. Dans ce cas, le bandeau n'est pas enroulé à l'aide du système de montage (10) décrit plus haut, mais est simplement vissé dans le filetage à partir de l'extrémité inférieure du corps de la râpe. Dans ce cas également, le bandeau est maintenu par encliquetage d'ergots dans des gorges (4, 5) du corps de la râpe.

Le pas (h) de l'hélice du filetage (3) est, par exemple, compris entre 1 mm et 5 mm. La largeur du bandeau (6) est, au maximum, égale au pas (h) de l'hélice, de façon à ce que les bords du bandeau soient, au plus, jointifs, mais ne se superposent pas.

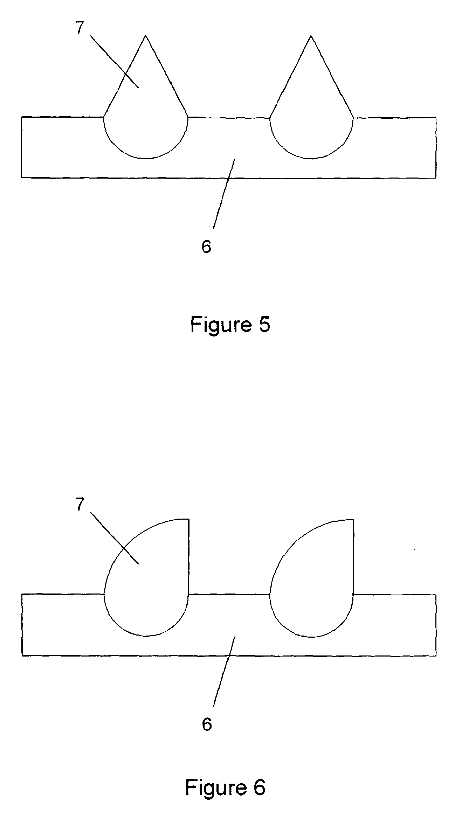

Chaque corps (1) de râpe peut accueillir des bandeaux munis d'éléments tranchants (7) plus ou moins agressifs. Ainsi, dans le mode de réalisation de la figure 3, les éléments tranchants (7) sont constitués, comme représenté sur la figure 3, de dents pyramidales présentant une ouverture (70) au niveau de leur sommet pour pouvoir libérer de la pression lors du râpage de façon à éviter une embolie graisseuse. Dans le mode de réalisation des figures 5 et 6, les éléments tranchants sont constitués d'arêtes faisant sensiblement toute la longueur du bandeau. Chacune des arêtes représentées sur la figure 5 présente deux plans de coupe, permettant de râper l'os à l'aller et au retour pendant le mouvement d'aller-retour de la râpe. En revanche, chacune des arêtes représentées sur la figure 6 ne présente qu'un seul plan de coupe, l'autre côté de l'arête étant arrondi. Ce dernier mode de réalisation ne permet donc de râper l'os qu'à l'aller ou au retour.

Il doit être évident, pour les personnes versées dans l'art, que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus. Ainsi, le bandeau peut être muni de tous types d'éléments tranchants habituellement utilisés pour râper un os.

## Revendications

1. Dispositif pour râper le canal intra-médullaire d'un os, comprenant un corps (1) allongé selon un axe sensiblement parallèle à l'axe longitudinal du canal et des éléments tranchants (7), le corps (1) allongé ayant une section transversale variable et une forme apte à coopérer avec la paroi interne du canal intra-médullaire de l'os, **caractérisé en ce que** les éléments tranchants (7) sont disposés sur un bandeau (6) amovible et jetable enroulé autour du corps (1) allongé suivant une rainure (3) hélicoïdale formée sur ledit corps (1).

2. Dispositif selon la revendication 1, **caractérisé en ce que** la rainure (3) est un filetage arrondi.

3. Dispositif selon la revendication 1 ou 2, **caractérisé en ce que** le bandeau (6) est souple, de façon à épouser la forme de la rainure (3), et **en ce qu'**il comprend au moins un ergot formé à proximité d'une extrémité du bandeau, ledit ergot coopérant avec une gorge (5) formée à proximité de l'extrémité supérieure de la rainure (3) pour maintenir le bandeau (6) en haut du corps (1).

4. Dispositif selon la revendication 3, **caractérisé en ce que** le bandeau comprend un deuxième ergot formé à proximité de l'autre extrémité du bandeau, ledit deuxième ergot coopérant avec une deuxième gorge (4) formée à proximité de l'extrémité inférieure de la rainure (3) pour maintenir le bandeau (6) en bas du corps (1).

5. Dispositif selon la revendication 3, **caractérisé en ce qu'**un manchon est vissé sur l'extrémité inférieure du corps (1) pour maintenir le bandeau (6) en bas du corps (1).

6. Dispositif selon une des revendications 1 à 5, **caractérisé en ce que** le bandeau (6) est collé sur le corps (1) grâce à une colle biocompatible.

7. Dispositif selon une des revendications 1 à 5, **caractérisé en ce que** le bandeau (6) est maintenu sur le corps (1) par un abrasif fixé sous le bandeau (6).

8. Dispositif selon une des revendications 1 à 5, **caractérisé en ce que** le bandeau (6) est un ressort vissé dans la rainure (3).

9. Dispositif selon une des revendications 1 à 8, **caractérisé en ce que** la largeur du bandeau (6) est, au maximum, égale au pas (h) de l'hélice, de façon à ce que, une fois que le bandeau (6) est rendu solidaire du corps (1), les bords du bandeau soient, au maximum, jointifs.

10. Dispositif selon une des revendications 1 à 9, **caractérisé en ce que** les éléments tranchants (7) du bandeau sont constitués d'une ou plusieurs lignes de dents.

11. Dispositif selon une des revendications 1 à 9, **caractérisé en ce que** les éléments tranchants (7) du bandeau sont constitués d'une ou plusieurs arêtes, de longueur sensiblement égale à la longueur du bandeau (6), chacune arête au moins un plan de coupe.

12. Système de montage (10) du bandeau (6) sur le corps (1) du dispositif selon une des revendications 1 à 7 et 9, **caractérisé en ce qu'**il comprend un axe (11) sensiblement horizontal pouvant tourner sur lui-même et mobile le long de son axe, sur lequel sont montés, l'un derrière l'autre, le corps (1) du dispositif, ainsi qu'un corps d'un deuxième dispositif servant de gabarit (12), les axes longitudinaux du corps du dispositif et du gabarit (12) étant sensiblement alignés avec ledit axe (11), et **en ce qu'**il comprend également une roue (13) sur laquelle est enroulé le bandeau (6), ainsi qu'un doigt (14), monté fixe en translation par rapport à la roue (13) et engagé dans le filetage du gabarit (12), la distance séparant le plan de la roue (13) et l'axe du doigt étant sensiblement égale à la distance séparant l'extrémité inférieure du corps (1) du dispositif de celle du gabarit (12), de telle sorte que, au fur et à mesure que le gabarit (12) et le corps (1) du dispositif subissent une rotation autour de l'axe (11) du système de montage (10), le doigt (14) se déplace le long du filetage du gabarit (12) et permet que le bandeau (6) s'enroule correctement dans le filetage du corps (1) du dispositif.

## Claims

1. Device for rasping the intramedullary canal of a bone, comprising a body (1) elongated along an axis substantially parallel to the longitudinal axis of the canal and cutting elements (7), the elongated body (1) having a variable cross section and a shape capable of co-operating with the internal wall of the intramedullary canal of the bone, **characterised in that** the cutting elements (7) are arranged on a removable, disposable strip (6) wound around the elongated body (1) along a helical groove (3) formed on said body (1).

2. Device according to Claim 1, **characterised in that** the groove (3) is a rounded threading.

3. Device according to Claim 1 or 2, **characterised in that** the strip (6) is flexible, in order to fit the shape of the groove (3), and **in that** it comprises at least one lug formed near one end of the strip, said lug co-operating with a throat (5) formed near the upper end of the groove (3) in order to hold the strip (6) at the top of the body (1).

4. Device according to Claim 3, **characterised in that** the strip comprises a second lug formed near the other end of the strip, said second lug co-operating with a second throat (4) formed near the lower end of the groove (3) in order to hold the strip (6) at the bottom of the body (1).

5. Device according to Claim 3, **characterised in that** a sleeve is screwed over the lower end of the body (1) in order to hold the strip (6) at the bottom of the body (1).

6. Device according to one of Claims 1 to 5, **characterised in that** the strip (6) is stuck to the body (1) using a biocompatible adhesive.

7. Device according to one of Claims 1 to 5, **characterised in that** the strip (6) is held on the body (1) by an abrasive fixed under the strip (6).

8. Device according to one of Claims 1 to 5, **characterised in that** the strip (6) is a spring screwed into the groove (3).

9. Device according to one of Claims 1 to 8, **characterised in that** the width of the strip (6) is equal at most to the pitch (h) of the helix, so that, once the strip (6) is made integral with the body (1), the edges of the strip are, at most, contiguous.

10. Device according to one of Claims 1 to 9, **characterised in that** the cutting elements (7) of the strip are constituted by one or more lines of teeth.

11. Device according to one of Claims 1 to 9, **characterised in that** the cutting elements (7) of the strip are constituted by one or more ridges, substantially equal in length to the length of the strip (6), each one ridge at least one cutting plane.

12. System (10) for mounting the strip (6) on the body (1) of the device according to one of Claims 1 to 7 and 9, **characterised in that** it comprises a substantially horizontal axis (11) capable of rotating on itself and mobile along its axis, on which are mounted, one behind the other, the body (1) of the device, as well as a body of a second device used as a jig (12), the longitudinal axes of the body of the device and the jig (12) being substantially aligned with said axis (11), and **in that** it also comprises a wheel (13) on which the strip (6) is wound, as well as a finger (14), mounted so as to be fixed in translation with respect to the wheel (13) and engaged in the threading of the jig (12), the distance separating the plane of the wheel (13) and the axis of the finger being substantially equal to the distance separating the lower end of the body (1) of the device from that of the jig (12), so that, as the jig (12) and the body (1) of the device undergo a rotation about the axis (11) of the mounting system (10), the finger (14) is displaced along the threading of the jig (12) and allows the strip (6) to be wound correctly within the threading of the body (1) of the device.

## Patentansprüche

1. Vorrichtung zum Raspeln des Knochenmarkskanals, mit einem Körper (1), der entlang einer zur Kanalachse im wesentlichen parallelen Achse länglich ist, und Schneidelementen (7), wobei der längliche Körper (1) einen veränderlichen Querschnitt und eine Form aufweist, die mit der Innenwand des Knochenmarkskanals zusammenwirken kann, **dadurch gekennzeichnet, dass** die Schneidelemente (7) an einem abnehmbaren Wegwerfband (6) angeordnet sind, das um den länglichen Körper (1) entlang einer am Körper (1) gebildeten schraubenförmigen Rille (3) gewickelt ist.

2. Vorrichtung nach Anspruch 1, **dadurch gekennzeichnet, dass** die Rille (3) eine abgerundetes Gewinde ist.

3. Vorrichtung nach Anspruch 1 oder 2, **dadurch gekennzeichnet, dass** das Band (6) biegsam ist, so dass es sich an die Form der Rille (3) anschmiegen kann, und dass es mindestens eine in der Nähe eines Endes des Bands gebildete Nase aufweist, wobei die Nase mit einer Nut (5), die in der Nähe des oberen Endes der Rille (3) gebildet ist, zusammenwirkt, um das Band (6) im oberen Teil des Körpers (1) zu halten.

4. Vorrichtung nach Anspruch 3, **dadurch gekennzeichnet, dass** das Band eine zweite, in der Nähe des anderen Endes des Bands gebildete Nase aufweist, wobei die zweite Nase mit einer zweiten Nut (4), die in der Nähe des unteren Endes der Rille (3) gebildet ist, zusammenwirkt, um das Band (6) im unteren Teil des Körpers (1) zu halten.

5. Vorrichtung nach Anspruch 3, **dadurch gekennzeichnet, dass** auf das untere Ende des Körpers (1) eine Muffe eingeschraubt ist, um das Band (6) im unteren Teil des Körpers (1) zu halten.

6. Vorrichtung nach einem der Ansprüche 1 bis 5, **dadurch gekennzeichnet, dass** das Band (6) mittels eines bioverträglichen Klebstoffs auf den Körper (1) geklebt ist.

7. Vorrichtung nach einem der Ansprüche 1 bis 5, **dadurch gekennzeichnet, dass** das Band (6) mittels eines unter dem Band (6) befestigten Schleifmittels am Körper (1) gehalten ist.

8. Vorrichtung nach einem der Ansprüche 1 bis 5, **dadurch gekennzeichnet, dass** das Band (6) eine in die Rille (3) eingeschraubte Feder ist.

9. Vorrichtung nach einem der Ansprüche 1 bis 8, **dadurch gekennzeichnet, dass** die Breite des Bands (6) höchstens gleich der Schrittwelle (h) der Spirale ist, so dass die Ränder des Bands höchstens bündig sind, wenn das Band (6) mit dem Körper (1) fest verbunden ist.

10. Vorrichtung nach einem der Ansprüche 1 bis 9 **dadurch gekennzeichnet, dass** die Schneidelemente (7) des Bands aus einer Reihe oder mehreren Reihen von Zähnen bestehen.

11. Vorrichtung nach einem der Ansprüche 1 bis 9 **dadurch gekennzeichnet, dass** die Schneidelemente (7) des Bands aus einer Kante oder mehreren Kanten bestehen, deren Länge im wesentlichen gleich der Länge des Bands (6) ist, wobei jede Kante mindestens eine Schneidebene aufweist.

12. Einrichtung zur Montage (10) des Bands (6) auf den Körper (1) der Vorrichtung nach einem der Ansprüche 1 bis 7 und 9, **dadurch gekennzeichnet, dass** sie eine im wesentlichen horizontale Achse (11) umfasst, die um sich selbst drehen kann und entlang ihrer Mittellinie beweglich ist, an der der Körper (1) der Vorrichtung sowie ein Körper einer zweiten Vorrichtung, die als Schablone dient, hintereinander angebracht sind, wobei die Längsachsen des Körpers der Vorrichtung und der Schablone (12) im wesentlichen mit der Achse (11) ausgerichtet sind, und dass sie auch ein Rad (13), auf welchem das Band (6) aufgewickelt ist, sowie einen Finger umfasst, der bezüglich des Rads (13) translatorisch fest verschiebbar ist und in das Gewinde der Schablone (12) eingreift, wobei der Abstand zwischen der Ebene des Rads (13) und der Achse des Fingers im wesentlichen gleich dem Abstand zwischen dem unteren Ende des Körpers (1) der Vorrichtung und demjenigen der Schablone (12) ist, so dass in dem Maße, wie die Schablone (12) und der Körper (1) der Vorrichtung eine Drehung um die Achse (11) der Montageeinrichtung (10) erfahren, sich der Finger (14) entlang des Gewindes der Schablone (12) verschiebt und es ermöglicht, dass das Band (6) sich einwandfrei in das Gewinde des Körpers (1) der Vorrichtung einwickelt.
